# EUROPEAN PATENT APPLICATION

(11) **EP 1 287 821 A1**
(43) Date of publication of application: **05.03.2003**
(21) Application number: 02255847.2
(22) Date of filing: 21.08.2002
(51) Int. Cl.: A61K 9/16, A61K 9/20, A61K 9/48, A61K 31/40, A61K 45/08, A61P 3/06

(54) **Multiparticulate formulations of atorvastatin calcium having a modulated rate of drug release**

(30) Priority: 04.09.2001 GB 0121436
(71) Applicant: Pfizer Limited, Sandwich, Kent CT13 9NJ (GB); PFIZER INC., New York, N.Y. 10017 (US)
(72) Inventor: Bilotte, Anne, c/o Pfizer Global Research and Dev, La Jolla, California 92037 (US)
(74) Representative: Wood, David John

(57) **Abstract**

The present invention is concerned with means for adjusting the bioavailability of atorvastatin calcium by modulating its rate of release from multiparticulate formulations and with multiparticulate formulations, especially tablets and capsules, having said modulated rate of release.

## Description

The present invention is concerned with means for adjusting the bioavailability of atorvastatin calcium by modulating its rate of release from multiparticulate formulations and with multiparticulate formulations, especially tablets and capsules, having said modulated rate of release.

Atorvastatin calcium is a selective competitive inhibitor of HMG-CoA having potent lipid lowering activity which is useful as a hypolipidaemic and/or hypocholesterolaemic agent. It is the subject of European Patent No. 0409281 and is currently sold under the name 'Lipitor™'.

European Patent No. 0680320 describes pharmaceutical compositions for the peroral treatment of hyperlipidaemia and hypercholesterolaemia which comprise atorvastatin calcium and at least one pharmaceutically acceptable metal salt additive designed to protect the active drug from any degrading or processing environment, as well as preserve it from photochemical decomposition during storage. A particularly preferred additive for this purpose is calcium carbonate. Such compositions typically comprise from 1% to 50% w/w of atorvastatin calcium and from 5% to 50% w/w of calcium carbonate and may be in the form of powders, tablets, dispersible granules, capsules, or cachets. There is no reference in the European patent to the pharmaceutically acceptable metal salt additives acting as biomodulators to alter the rate of release of atorvastatin calcium and therefore its bioavailability.

The term 'biomodulator' means a substance used in a formulation which has an effect on the release rate of the active agent and thus can be used to regulate its bioavailability. Biomodulators can have a positive effect, that is, their presence may serve to increase the rate of release and therefore the bioavailability of the active agent or, as in the present invention, they may have a negative effect in that their presence suppresses the rate of release and therefore the bioavailability of the active agent. By using an appropriate amount of a suitable biomodulator, it is possible to optimise the rate of release and bioavailability of the active agent.

In the non-multiparticulate formulations of European Patent No. 0680320, the presence of a pharmaceutically acceptable metal salt additive has been found to have a positive biomodulating effect in that increasing its content serves to increase the rate of release and bioavailability of the atorvastatin calcium. Unfortunately, the effect on stability of reducing the calcium carbonate content mitigates against the use of calcium carbonate as a biomodulator in such formulations. In other words, the bioavailability of the atorvastatin calcium is maximised in the interests of stability.

Surprisingly, we have now found that, in contrast to non-multiparticulate formulations, multiparticulate formulations comprising atorvastatin calcium and calcium carbonate have a relatively poor rate of release and bioavailability. On the other hand, formulations lacking calcium carbonate not only remain stable, but exhibit a very similar rate of release and bioavailability to non-multiparticulate formulations containing calcium carbonate, for example, the commercially available tablet. Thus the calcium carbonate is still behaving as a biomodulator, but unexpectedly in a reverse sense to that observed in non-multiparticulate formulations. This dichotomy in the behaviour of calcium carbonate between multiparticulate and non-multiparticulate formulations has yet to be satisfactorily explained.

Using the multiparticulate formulations of the invention, it is possible by adjusting the loading of calcium carbonate to both retain stability *and* modulate the rate at which atorvastatin calcium is released. By judicious inclusion of the correct amount of calcium carbonate, it is possible for the first time to provide a formulation having a rate of release which is less than that of the non-particulate formulations described in the prior art and a bioavailability specifically suited to the needs of the patient. In other words, by using a multiparticulate formulation in accordance with the invention, the rate of release and bioavailability of atorvastatin calcium may be optimised for the particular patient undergoing treatment.

According to the present invention, therefore, there is provided means for adjusting the bioavailability of atorvastatin calcium by modulating its rate of release from multiparticulate formulations. Specifically, there are provided (a) a multiparticulate formulation having *comparable* bioavailability to a non-multiparticulate CaCO₃-containing formulation which formulation comprises atorvastatin calcium and a pharmaceutically acceptable carrier, diluent or excipient which is not calcium carbonate and (b) a multiparticulate formulation having *optimised* bioavailability which additionally comprises a biomodulator. A preferred biomodulator for the purposes of the invention is calcium carbonate. The use of both formulations as medicaments for the treatment of hyperlipidaemia and hypercholesterolaemia are also provided.

Tablets in accordance with the invention may contain carriers, such as calcium carbonate, dibasic calcium phosphate, glycine, lactose, mannitol, microcrystalline cellulose, sodium citrate and starch (preferably corn, potato or tapioca starch), disintegrants, such as croscarmellose sodium, sodium starch glycollate and certain silicates, and granulation binders, such as acacia, bentonite, gelatin, hydroxypropylcellulose, hydroxypropylmethylcellulose (HPMC), polyvinylpyrrolidone, sorbitol, sucrose and triglycerides. Lubricating agents, such as glyceryl behenate, magnesium stearate, PEG, stearic acid and talc, wetting agents, such as sodium lauryl sulphate, anti-oxidants, colourants, flavours and preservatives may also be present.

The tablets of the invention may be manufactured by any standard process, for example, by direct compression, granulation (wet, dry, or melt), melt congealing and extrusion. The tablet cores may be mono- or multilayered and may be coated or left uncoated.

Similar compositions may be employed as fillers in the capsules of the invention. Gelatin, HPMC and starch capsules are particularly suitable for this purpose. Preferred carriers include cellulose, high MW polyethylene glycols, lactose and starch.

### FIGURES

Figure 1 illustrates the disparity in release rates between the CaCO₃-containing multiparticulate formulation of Example 1 and a commercial Lipitor™ tablet containing 30% by weight of CaCO₃.

Figure 2 illustrates the disparity in bioequivalence between the CaCO₃-containing multiparticulate formulation of Example 1 and a commercial Lipitor™ tablet containing 30% by weight of CaCO₃.

Figure 3 illustrates the similarity in the release rates of the CaCO₃-free multiparticulate formulation of Example 2 and a commercial Lipitor™ tablet containing 30% by weight of CaCO₃.

Figure 4 illustrates the similarity in bioequivalence of the CaCO₃-free multiparticulate formulation of Example 2 and a commercial Lipitor™ tablet containing 30% by weight of CaCO₃.

### EXAMPLES

Uniformly blended material suitable for the preparation of multiparticulate formulations according to the invention may be obtained by any of the processes described in the examples. The resulting material is extruded and spheronised to give pellets which may be filled into a capsule or compressed into a tablet.

### EXAMPLE 1 (COMPARATIVE)

### PREPARATION OF ATORVASTATIN CALCIUM MULTIPARTICULATE FORMULATION COMPRISING 10% ATORVASTATIN CALCIUM, 30% CaCO₃, AVICEL PH101/LACTOSE 110M (1:1) AND 5% AcDiSol

### Protocol

A routine blend/screen method was used. The ingredients were weighed directly into a suitable container and blended for 15 minutes using a Turbula mixer. The resulting blend was screened through a 500µm mesh sieve. Blending and screening were repeated followed by a final 15-minute blend.

The homogeneity of the blend was checked by HPLC and a % potency of 98-102% achieved with RSD <5%.

The blend was wet massed with water or 0.1M Tris buffer solution until, when compressed, the blend broke cleanly. It was then gradually fed into a small-scale extruder (Caleva Model 15) and the extrudate collected. The extrudate was spheronised (Caleva Model 250) at about 600rpm for 5-10 minutes. The resulting pellets were transferred to a tray and dried in an oven at 50°C overnight. Finally, these pellets can be either filled into a capsule or compressed into a tablet.

The tablets had a tap density of approximately 10% and approximately 97% were in the size range 500-1200µm.

### Results

The release profile of atorvastatin calcium from the multiparticulate systems was assessed by two methods:

### (a) Release Rate

Pharmacopoeia method in sink conditions using phosphate buffer at pH 6.8 to determine % release of atorvastatin calcium from multiparticulates:
Baskets: 100rpm
Media: 0.05M potassium dihydrogen orthophosphate adjusted to pH 6.8 using 10M potassium hydroxide (900ml/vessel)
Temperature: 37°C
Pathlength: 1cm
Wavelength: 244nm
E₁¹: 399
Sample weight: 400mg in capsules

### Result: Approximately 100% release within 15 minutes

### (b) Bioequivalence

Non-sink method using acetate buffer at pH 4.5 to discriminate between bioequivalent and non-bioequivalent formulations of atorvastatin calcium:
Paddles: 50rpm
Media: 0.05M sodium acetate adjusted to pH 4.5 using HCI (500ml/vessel)
Temperature: 37°C
Pathlength: 1cm
Wavelength: 244nm
E₁¹: 399
Sample weight: 400mg in capsules

### Conclusion

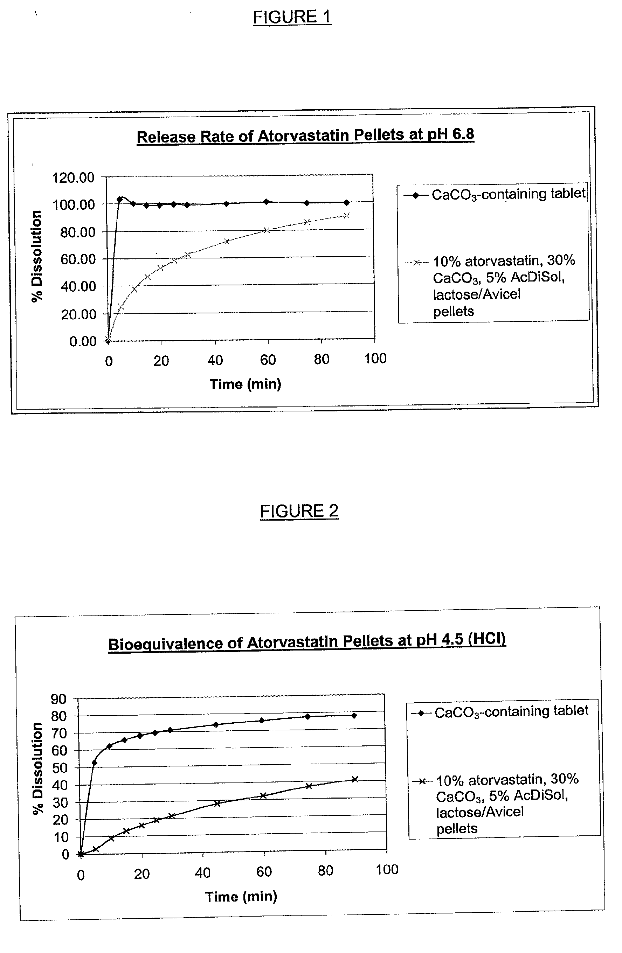

As shown in Figures 1 and 2, the release profile of the CaCO₃-containing multiparticulate formulation of Example 1 was significantly inferior to that of a CaCO₃-containing tablet, *i.e.* the CaCO₃-containing multiparticulate formulation of Example 1 was not bioequivalent to a CaCO₃-containing tablet in accordance with the prior art.

### EXAMPLE 2

### PREPARATION OF ATORVASTATIN CALCIUM MULTIPARTICULATE FORMULATION COMPRISING 10% ATORVASTATIN CALCIUM, AVICEL PH101/LACTOSE 110M (1:1) AND 15% AcDiSol

### Protocol

The multiparticulate formulation of Example 2 was prepared according to the process of Example 1; the CaCO₃-free blend was found to be easier to extrude/spheronise than the CaCO₃-containing blend of Example 1.

### Results

As before, the release profile of atorvastatin from the multiparticulates was assessed using the methods described in Example 1.

### Conclusion

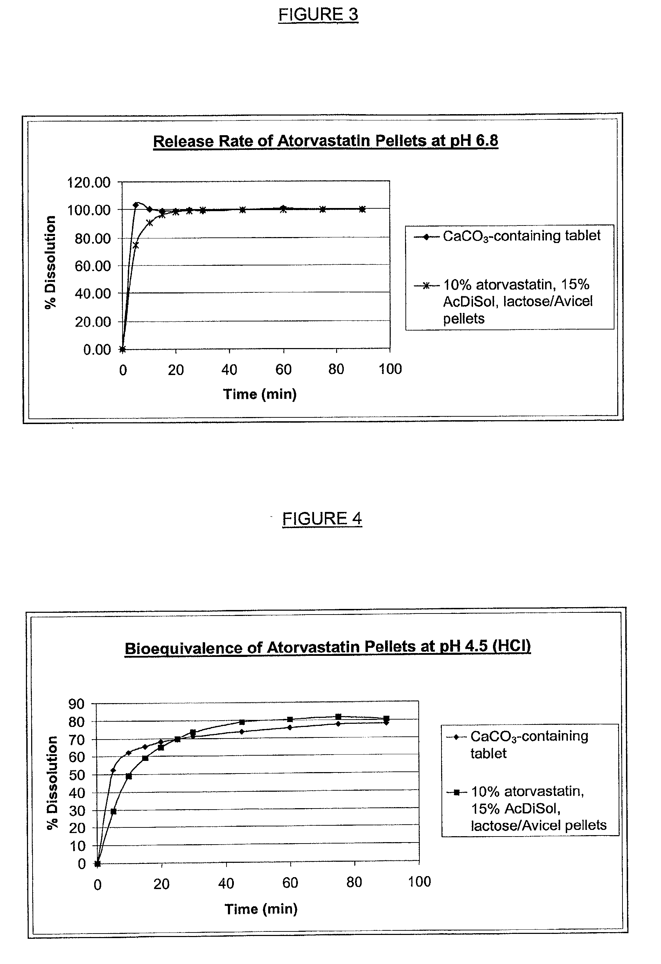

As shown in Figures 3 and 4, the release profile of the CaCO₃-free multiparticulate formulation of Example 2 was very similar to that of a CaCO₃-containing tablet, *i.e.* the CaCO₃-free multiparticulate formulation of Example 2 was unexpectedly found to be bioequivalent to a CaCO₃-containing tablet in accordance with the prior art.

### Summary

The CaCO₃-containing multiparticulate formulation of Example 1 was difficult to extrude/spheronise, showed a poor rate of release of atorvastatin calcium (only about 40% after 10 minutes) and was not bioequivalent to a CaCO₃-containing tablet. By omitting the CaCO₃ component (Example 2), it was unexpectedly found possible to produce a multiparticulate formulation having a rate of release and bioequivalence very similar to those of the commercial CaCO₃-containing tablet. It follows that by including an appropriate amount of CaCO₃ in the multiparticulate formulation of Example 2, it would be possible to reduce the rate of release of atorvastatin calcium and its bioavailability to suit the needs of individual patients.

## Claims

1. A multiparticulate formulation comprising atorvastatin calcium and a pharmaceutically acceptable carrier, diluent or excipient other than calcium carbonate.

2. A multiparticulate formulation according to Claim 1, which formulation additionally comprises a biomodulator.

3. A multiparticulate formulation according to Claim 2, wherein said biomodulator is calcium carbonate.

4. A multiparticulate formulation according to any of Claims 1 to 3, which formulation is in the form of a tablet or capsule.

5. A multiparticulate formulation according to any of Claims 1 to 4 for use as a medicament.

6. The use of atorvastatin calcium in the manufacture of a medicament, which medicament is in the form of a multiparticulate formulation for oral administration in the treatment of hyperlipidaemia.

7. The use of atorvastatin calcium in the manufacture of a medicament, which medicament is in the form of a multiparticulate formulation for oral administration in the treatment of hypercholesterolaemia.

8. The use of atorvastatin calcium combined with a biomodulator in the manufacture of a medicament, which medicament is in the form of a multiparticulate formulation for oral administration in the treatment of hyperlipidaemia.

9. The use of atorvastatin calcium combined with a biomodulator in the manufacture of a medicament, which medicament is in the form of a multiparticulate formulation for oral administration in the treatment of hypercholesterolaemia.

10. Use according to Claim 8 or 9, wherein said biomodulator is calcium carbonate.

11. Use according to any of Claims 6 to 10, wherein said medicament is in the form of a tablet or capsule.
